# FASCICULE DE BREVET EUROPEEN

(11) **EP 3 031 925 B1**
(45) Date de publication et mention de la délivrance du brevet: **07.07.2021**
(21) Numéro de dépôt: 15189670.1
(22) Date de dépôt: 14.10.2015
(51) Int. Cl.: C12P 7/64, A61K 8/92, A61Q 19/00, A61K 8/06

(54) **PROCÉDÉ DE PRÉPARATION D'UN EXCIPIENT À BASE DE CIRE PAR CATALYSE ENZYMATIQUE**
VERFAHREN ZUR HERSTELLUNG EINES TRÄGERSTOFFS AUF WACHSBASIS DURCH ENZYMATISCHE KATALYSE
METHOD FOR PREPARING A WAX-BASED CARRIER BY ENZYMATIC CATALYSIS

(30) Priorité: 10.12.2014 FR 1462153
(43) Date de publication de la demande: 15.06.2016
(73) Titulaire: GATTEFOSSE SAS, 69804 Saint-Priest Cedex (FR)
(72) Inventeur: RODIER, Jean-David, 69100 Villeurbanne (FR); BOULGHOBRA, Hakime, 69330 Meyzieu (FR)
(74) Mandataire: Cabinet Laurent & Charras

(56) Documents cités:
- FR-A1- 2 891 736
- US-A- 4 659 514
- US-A- 5 219 733
- US-A1- 2009 181 439
- GUNAWAN E R ET AL: "Study on response surface methodology (RSM) of lipase-catalyzed synthesis of palm-based wax ester", ENZYME AND MICROBIAL TECHNOLOGY, STONEHAM, MA, US, vol. 37, no. 7, 1 décembre 2005 (2005-12-01), pages 739-744, XP027765887, ISSN: 0141-0229 [extrait le 2005-12-01]
- MUKHERJEE K D ET AL: "PREPARATION OF ESTERS RESEMBLING NATURAL WAXES BY LIPASE-CATALYZED REACTIONS", JOURNAL OF AGRICULTURAL AND FOOD CHEMISTRY, AMERICAN CHEMICAL SOCIETY, US, vol. 36, no. 6, 1 novembre 1988 (1988-11-01), pages 1333-1336, XP000024578, ISSN: 0021-8561, DOI: 10.1021/JF00084A052
- GEORG STEINKE ET AL: "High-Yield Preparation of Wax Esters via Lipase-Catalyzed Esterification Using Fatty Acids and Alcohols from Crambe and Camelina Oils", JOURNAL OF AGRICULTURAL AND FOOD CHEMISTRY, vol. 49, no. 2, 1 février 2001 (2001-02-01), pages 647-651, XP055195654, ISSN: 0021-8561, DOI: 10.1021/jf000852i
- Anonymous: "Cire d'abeille - Wikipédia", , 25 March 2020 (2020-03-25), pages 1-5, XP055679756, Retrieved from the Internet: URL:https://fr.wikipedia.org/wiki/Cire_d'a beille [retrieved on 2020-03-25]
- THOMAS K MIWA: "Jojoba Oil Wax Esters and Derived Fatty Acids and Alcohols: Gas Chromatographic Analyses", JOURNAL OF THE AMERICAN OIL CHEMISTS' SOCIETY (JAOCS), SPRINGER, DE, vol. 48, no. 6, 1 June 1971 (1971-06-01), pages 259-264, XP007920125, ISSN: 0003-021X, DOI: 10.1007/BF02638458
- Joel Chopineau ET AL: "Production of biosurfactants from sugar alcohols and vegetable oils catalyzed by lipases in a nonaqueous medium", Biotechnology and Bioengineering, vol. 31, no. 3, 20 February 1988 (1988-02-20), pages 208-214, XP055077835, ISSN: 0006-3592, DOI: 10.1002/bit.260310305

## Description

### DOMAINE DE L'INVENTION

La présente invention concerne l'utilisation comme excipient d'un produit obtenu par modification de cires par des polyols, par catalyse enzymatique telle que définie dans les revendications.

Le domaine d'utilisation de la présente invention concerne entre autres l'industrie de la cosmétique et des produits de soin personnel et l'industrie pharmaceutique. En pratique, l'excipient de l'invention peut être utilisé en particulier aussi bien pour son effet émulsionnant qu'émollient et texturant.

### ETAT ANTERIEUR DE LA TECHNIQUE

Les cires sont largement utilisées dans l'industrie cosmétique, en particulier comme agent durcissant dans les rouges à lèvres ou comme agent épaississant dans certaines émulsions. Leur composition particulièrement complexe, est constituée d'un mélange de monoesters d'acides et d'alcools gras à très longues chaînes, d'acides gras libres et d'alcools gras libres parfois différents des acides et des alcools estérifiés, d'hydrocarbures, d'hydroxyesters et d'autres composés comme les dérivés terpéniques.

De nombreux travaux ont été réalisés pour mettre au point des produits substituts de cires. Ces substituts comportent essentiellement des esters aliphatiques d'acides et d'alcools gras.

De manière générale, les procédés de transformation appliquée aux produits de l'oléochimie, notamment dans la synthèse d'esters gras se font par les voies chimiques classiques ou par voie enzymatique.

Les procédés par catalyse enzymatique sont particulièrement intéressants par rapport aux procédés chimiques. En effet, ils permettent de réaliser des synthèses à des températures bien inférieures à celles employées pour la voie chimique classique et ainsi d'économiser de l'énergie. En outre, les matières premières subissent moins de dégradations et cela permet d'éviter des étapes supplémentaires de purification comme la décoloration ou la désodorisation (Thum et al., SOFW-Journal, 2008, 134, 43-47).

Dans cette optique d'amélioration de la qualité des produits et de limitation de l'impact énergétique et des déchets générés par les procédés, les émollients suivants ont été produits par catalyse enzymatique : myristate de myristyle, cocoate de décyle, ricinoléate de cétyle, éruçate d'oléyle et cocoate d'isoamyl (Ansorge-Schumacher, 2013, Chem. Soc. Rev., 42, 6475).

Les lipases forment une famille d'enzymes qui sont naturellement capables d'hydrolyser les liaisons esters. Elles ont aussi la capacité de catalyser des réactions d'estérification, d'alcoolyse et d'interestérification, c'est-à-dire de redistribution des acides gras, en milieux non-aqueux (Zhang et al., J. Am. Oil. Chem. Soc., 2001, 78(1), 57-64).

Ainsi, on retrouve dans la littérature, plusieurs documents se rapportant à la synthèse enzymatique d'ester gras. Dans la plupart des cas, les acides gras et alcools gras servant à la préparation des esters sont limités de par leur chaîne carbonée à 18 carbones. On peut citer à titre d'exemple :
- la préparation de l'ester 2-éthylhexanoate de cétyle (Chen et al., J. Am. Oil Chem. Soc., 2011, 88(12), 1917-1923) ;
- la synthèse d'esters d'alcool oléique avec des acides gras allant de l'acide laurique (12 carbones) aux acides stéarique, oléique et linoléique (18 carbones) (Gunawan et al., Journal of Oleo Science, 2004, 53(10), 471-477) ;
- la préparation d'oléate d'oléyle, utilisé comme substitut de la cire de jojoba (*simmondsia chinensis*), à partir de trioléine et d'alcool oléique (Hadzir et al., 2001, 76(5), 511-515) ;
- la synthèse d'esters à partir d'acide oléique et l'alcool oléique (Radzi et al., Journal of Oleo Science, 2010 59(3), 127-134) ;
- la production d'esters de décanol et d'acide hydroxylé comme le 9-hydroxymethyloctadécène-10-oïque (Multzsch et al., J. Am. Oil Chem. Soc., 1994, 71(7), 721-725) ;
- la synthèse de l'ester palmitate d'oléyle (Yasin et al., Biosciences Biotechnology Research Asia, 2007, 4(1), 59-64).

D'autres études font référence à des alcools et des acides à très longues chaînes, c'est-à-dire au-delà de 18 carbones. On peut citer par exemple :
- la synthèse d'esters de policosanols, en faisant réagir des alcools gras dont la chaîne comprend 24 à 34 carbones, avec de l'acide linoléique conjugué (Zhao et al., Biocatalysis and Biotransformation, 2013, 31(2), 114-122) ;
- la préparation de béhénate de béhényle en partant de l'acide béhénique (C22 :0) et de l'alcool béhénique (Petersson et al., Green Chemistry, 2005(7), 837-843) ;
- la préparation d'esters à chaînes possédant de 32 à 46 carbones, à partir d'alcools et acides gras à longues chaînes (de 18 à 24 carbones) obtenus par transformation de l'huile de crambe ou de cameline (Steinke et al., Journal of Agricultural and Food Chemistry, 2001, 49(2), 647-651).

Les esters à longues chaînes peuvent aussi être préparés à partir d'alcools gras et d'esters méthyliques d'acides gras (Ucciani et al., Fett/Lipid, 1996, 98(6), 206-210).

On trouve d'autres exemples de synthèses d'esters mettant en œuvre des acides gras de longueurs inférieures comme les acides palmitique et stéarique avec l'alcool oléique (Kapucu et al., Chemical Engineering Communications, 2003, 190(5-8), 779-796 ou Guncheva et al., Biotechnology Letters, 2008, 30(3), 509-512).

Dans d'autres synthèses d'esters, les acides gras peuvent être associés à des polyols. Ainsi la réaction entre l'acide stéarique et le glycérol a été décrite (Blanco et al., Industrial and Engineering Chemistry Research, 2009, 48(15), 6957-6960).

Une autre synthèse met en œuvre l'acide ricinoléique qui réagit avec le triméthylol propane, le pentaérythritol ou le dimère diol dans le but de constituer des polymères (Kelly et al., Journal of applied polymer science, 2006, 101(3), 1646-1656).

Dans un autre exemple, le document US 2009/0181439 décrit une réaction d'estérification entre polyols et acides gras. Le polyéthylène glycol ou le polypropylène glycol réagissent avec l'acide undécène-10-oïque en présence d'une lipase. Ce document décrit également la production de myristate de myristyle par un procédé en continu.

Le document US 5 219 733 A décrit un procédé visant à faire réagir un alcool primaire ou secondaire avec un acide gras ou un ester d'acide gras et ce, en présence d'une lipase.

Le document Mukherjee et al., 1988 (Journal of Agricultural and Food Research, 36(6): 1333-1336) décrit la préparation d'un ester par alcoolyse ou par estérification, en présence d'une lipase.

Dans un système biphasique et sans solvant, la lipase *candida antarctica* est utilisée pour catalyser la réaction d'estérification entre l'acide laurique et le polyglycérol (Hilterhaus et al., Organic Process Research & Development, 2008, 12(4), 618-625).

De manière générale, les procédés utilisés pour réaliser les réactions d'estérification, de transestérification ou d'interestérification catalysées par des enzymes peuvent être en discontinu, c'est-à-dire en réacteur agité (voir notamment Blanco et al., Industrial and Engineering Chemistry Research, 2009, 48(15), 6957-6960 ; Otero et al., J. Am. Oil Chem. Soc., 2013, 90, 81-90; Steinke et al., Journal of Agricultural and Food Chemistry, 2001, 49(2), 647-651 ; Yasin et al., Biosciences Biotechnology Research Asia, 2007, 4(1), 59-64 ; Zhang et al., J. Am. Oil Chem. Soc., 2001, 78(1), 57-64).

De même, ces procédés peuvent être des procédés continus, c'est-à-dire constitués d'un lit fixe de catalyseur à travers lequel les produits à transformer circulent en boucle (voir notamment Huey et al., Journal of Food Science, 2009, 74(4), E177-E183 ; FR 2 930 782).

Le document US 2010/0167360 propose par exemple un procédé continu pour la synthèse d'esters en partant d'acides gras.

Dans les deux cas de figure, le catalyseur enzymatique est idéalement supporté sur une résine polymère. Les techniques d'immobilisation des catalyseurs enzymatiques sur des supports sont bien connues dans l'état de la technique (Alloue et al., Biotechnol. Agron. Soc. Environ., 2008, 12(1), 57-68).

La température employée pour réaliser ces réactions d'estérification, de transestérification ou d'interestérification est déterminée par la nature des réactifs. En effet, les réactions de transformation se font sur des produits liquides. Les cires bien souvent solides à température ambiante, devront donc être fondues.

Par conséquent, la température peut varier de la température ambiante, comme pour les produits fortement insaturés, à la température de fusion des esters à longues chaînes ou des acides et des alcools de départ.

Ainsi une étude décrit la synthèse enzymatique du béhénate de béhényle par réaction d'estérification où la température nécessaire pour obtenir un produit liquide est de 88°C (Petersson et al., Green Chemistry, 2005(7), 837-843).

La gamme d'enzymes utilisables dépend aussi bien des réactifs, c'est-à-dire de la nature des acides, esters et alcools que l'on veut faire réagir que de l'état physique du milieu réactionnel.

Comme il a déjà été souligné, les cires sont des produits largement employés dans l'industrie, notamment dans l'industrie cosmétique.

Outre les procédés de synthèse de substituts de cires (esters gras), à partir d'acides et d'alcool gras, la transformation des cires, qu'elles soient naturelles ou artificielles, est particulièrement intéressante en ce qu'elle mène à des produits possédant de nouvelles propriétés (Lennon et al., Cosmetics & Toiletries, 2010, 125(1), 38-43).

Bien que la transformation de cires naturelles par voie chimique en présence de polyols ait déjà été décrite, ces réactions se font à des températures relativement élevées, en présence d'un catalyseur chimique comme un hydroxyde de métal alcalin.

A titre d'exemple, le document FR 2 542 008 décrit la réaction de la cire d'abeille avec du PEG (polyéthylèneglycol) à une température comprise entre 180 et 240°C et en présence d'un catalyseur alcalin comme l'hydroxyde de sodium. Le produit résultant de cette réaction est notamment utilisé pour préparer des émulsions.

Le document EP 0 319 062 présente la dérivation des acides gras libres de la cire d'abeille par du glycidol, à une température comprise entre 80 et 150°C, idéalement en présence d'un catalyseur basique. Le produit obtenu contient des esters de glycerol, voire des esters de polycondensat de glycidol. Ce produit est notamment utilisé pour préparer des émulsions et des gels.

Le document FR 2 891 736 décrit la transformation d'un mélange de cire liquide et de cire solide, en présence d'un polyol et d'un catalyseur, de préférence alcalin, à une température comprise entre 150 et 200°C.

Quoi qu'il en soit, l'ensemble des documents cités ci-dessus font état de réactions de transformation de cires par voie chimique, conduites à des températures de réaction élevées, se situant bien au-delà de la température de fusion des cires, et en présence d'un catalyseur chimique.

Dans de telles conditions, les cires peuvent subir les effets de la chaleur pendant la réaction, même sous atmosphère protectrice (sous azote par exemple).

Ces effets peuvent se traduire par une modification de la couleur et de l'odeur, les cires peuvent ainsi s'assombrir et donner au produit final une odeur plus forte. En effet, les cires végétales telles que les cires de son de riz, de candelilla ou de carnauba peuvent gagner en coloration lorsqu'elles sont chauffées à une température élevée, c'est-à-dire à plusieurs dizaines de degrés au-delà de leur température de fusion.

Dans les cas de transformation des cires à haute température, des étapes de purification comme la désodorisation ou la décoloration sont nécessaires pour retrouver des valeurs correspondant aux standards utilisés dans les applications cosmétiques.

Ainsi, pour la préparation d'excipients par réaction entre une cire et un polyol, les procédés par voie chimique classiques de l'art antérieur présentent l'inconvénient de modifier la couleur de la cire. Par conséquent, l'excipient est généralement purifié et traité pour éliminer cette coloration néfaste.

Afin de remédier à ce problème, le Demandeur a mis au point un procédé de préparation d'un excipient ne nécessitant pas de traitement de décoloration ni de désodorisation.

### EXPOSE DE L'INVENTION

La présente invention concerne l'utilisation d'un excipient dans une formulation cosmétique ne présentant pas de problème de coloration résultant des conditions expérimentales de la réaction mise en œuvre.

Plus précisément, le procédé de préparation d'un excipient est effectué par réaction d'au moins une cire avec au moins un polyol en présence d'au moins une enzyme.

L'enzyme mise en œuvre dans le procédé fait avantageusement partie de la famille des lipases. Elle est avantageusement choisie dans le groupe comprenant : *candida antarctica lipase A ; candida antarctica lipase B ; candida rugosa ; thermomyces lanuginosus ; rhizomucor miehei ; pseudomonas sp.* ; et *carica papaya.*

De préférence, l'enzyme utilisée est *candida antarctica lipase B.*

Selon un autre mode de réalisation préférentiel, l'enzyme peut être supportée sur un substrat solide. Le support de l'enzyme sur le substrat se fait de manière covalente ou non covalente.

De manière avantageuse, le substrat solide est une résine ou un polymère pouvant notamment être choisi dans le groupe comprenant : polyacrylate ; polyméthacrylate ; polyvinylstyrène ; copolymères styrène-divinylbenzène ; polypropylène ; polyéthylène ; polyéthylène téréphtalate ; polytétrafluoroéthylène ; polyacrylamide ; polyamide ; les mélanges de ces polymères ; les copolymères des monomères constituant ces polymères ; polysaccharides ; protéines ; bentonite ; silice ; les supports oxydes ; et les céramiques.

A titre d'exemple, le substrat solide supportant l'enzyme peut notamment être tel que décrit dans le document US 2010/0167360. L'homme du métier saura donc mettre en œuvre les techniques nécessaires à la réalisation d'une enzyme supportée.

De manière générale la cire peut être naturelle ou bien synthétique. Le terme cire naturelle désigne toute cire d'origine naturelle c'est-à-dire d'origine végétale, animale ou minérale comme la cire de lignite.

L'état de l'art définit les cires naturelles comme un mélange complexe qui comprend généralement des alcools gras et des acides gras à longues chaînes carbonées, des esters d'alcools et d'acides gras, voire d'hydroxyacides à longues chaînes hydrocarbonées, des triterpènes et leurs esters, ainsi que des esters d'acides contenant dans leurs chaînes au moins un noyau aromatique.

De manière générale, les esters (RC(=O)O-R¹) constituant les cires naturelles comprennent des chaînes carbonées, avantageusement hydrocarbonées, R¹ d'au moins 20 atomes de carbone, et plus avantageusement 20 à 32 atomes de carbone. R, avantageusement un groupement hydrocarboné, possède au moins 16 atomes de carbone comme dans la cire d'abeille et avantageusement 20 à 32 atomes de carbone.

Le terme cire synthétique désigne toute cire préparée d'une manière artificielle, utilisant les voies de synthèse en chimie.

Les cires synthétiques comprennent généralement un mélange d'esters d'acides gras et d'alcools gras obtenus par estérification, avec R et R¹ compris avantageusement entre 12 et 30 atomes de carbones.

De manière générale, l'enzyme utilisée dans le procédé peut être toute enzyme capable de catalyser la réaction de modification entre une cire et un polyol. Il s'agit plus particulièrement des réactions suivantes :
- transestérification, c'est-à-dire l'échange du groupement R¹ d'un ester RC(=O)O-R¹ avec le groupement R² d'un alcool R²OH ;
- estérification, c'est-à-dire réaction entre un acide et un alcool ;
- interestérification, c'est-à-dire de redistribution des acides gras des esters avec d'autres alcools gras.

L'enzyme permet de catalyser ces trois types de réactions simultanément étant donné qu'une cire est un mélange contenant des esters, des acides et des alcools.

Selon un mode de réalisation avantageux, la cire est une cire naturelle qui peut être avantageusement choisie dans le groupe comprenant : cire d'abeille ; cire de candelilla ; cire de carnauba ; cire de tournesol ; cire de son de riz ; cire de canne à sucre ; cire de jojoba ; cire de mimosa ; cire d'olive ; et cire de shellac.

Un mélange de cires peut également être utilisé.

De manière plus avantageuse, la cire naturelle est la cire d'abeille.

La composition et la température de fusion de certaines des cires naturelles pouvant être utilisées sont présentées dans le tableau suivant :

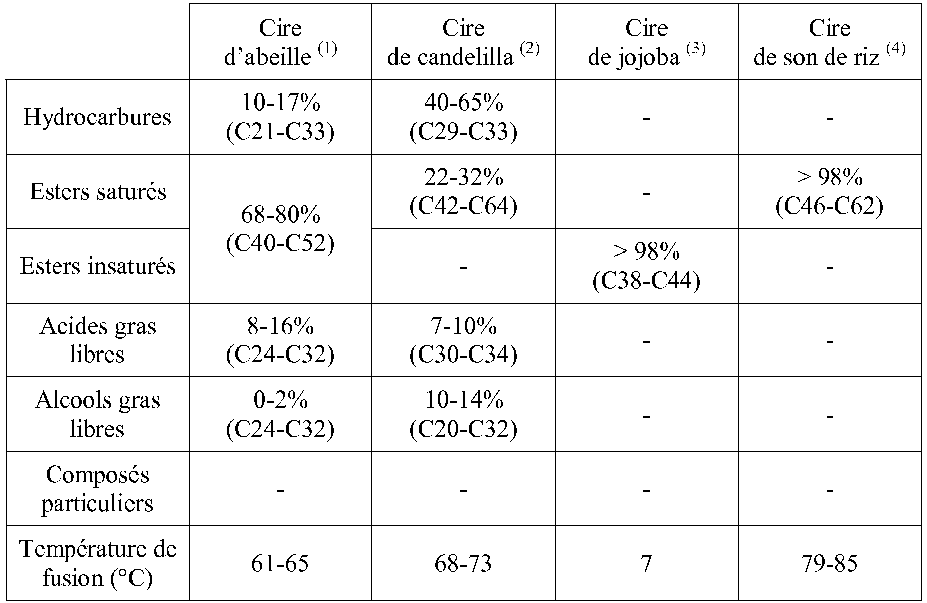

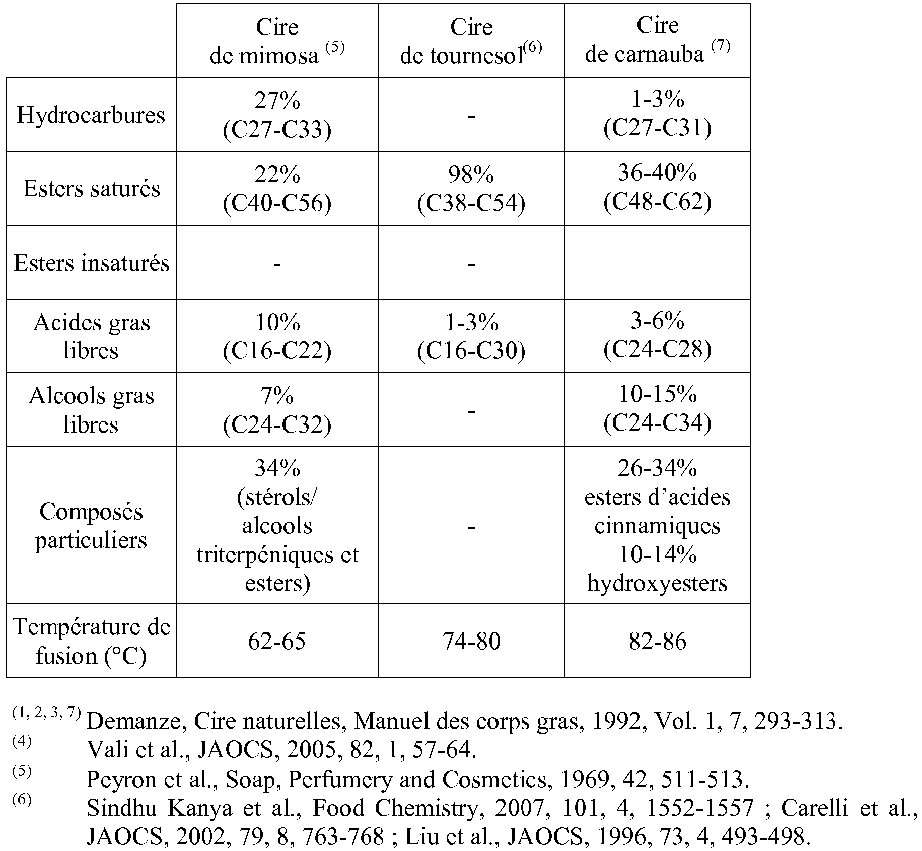

Les pourcentages sont exprimés en masse. La longueur des chaînes hydrocarbonées (C38-C54 par exemple) correspond aux groupements R+R¹ pour les esters (RC(=O)O-R¹) ou (C24-C32) correspond au groupement R¹ des alcools R¹-OH ou R³ des acides R³C(=O)-OH.

Le terme polyol désigne une molécule, notamment une chaîne carbonée linéaire ou ramifiée pouvant contenir des hétéroatomes, comprenant au moins deux groupements hydroxyles (-OH). De manière encore plus avantageuse, il comprend 2 à 7 groupements hydroxyles, et encore plus avantageusement 3 à 5.

Le polyol utilisé dans le procédé peut être avantageusement choisi dans le groupe comprenant : éthylène glycol ; diéthylène glycol ; triéthylène glycol ; polyéthylène glycol ; 2-méthyl propanediol ; propylène glycol ; polypropylène glycol ; butylène glycol ; néopentyl glycol ; hexylène glycol ; octylène glycol ; triméthylol propane ; sorbitol ; érythritol ; pentaerythritol ; dipentaerythritol ; glycérol ; diglycérol ; polyglycérol ; isosorbide ; sorbitan ; polytriméthylène éther glycol; et 1,3-propanediol. Un mélange de polyols peut également être utilisé.

De manière plus avantageuse, le polyol est le polyglycérol (PGly). Il peut notamment s'agir du polyglycérol-3 (PGly-3) correspondant au numéro CAS 25618-55-7.

Le polyglycérol peut notamment se présenter sous la forme d'un mélange de di-, tri-, tétra- et pentaglycérol, soit entre 2 et 5 unités répétitives glycérol comportant respectivement 4 à 7 fonctions OH.

La masse molaire du PGly est avantageusement comprise entre 166 et 388 g/mol.

Selon un autre mode de réalisation, la réaction est réalisée également en présence d'au moins un acide gras, avantageusement de formule R³C(=O)-OH, R³ étant un groupement hydrocarboné comprenant 16 à 24 atomes de carbone. L'acide gras peut contenir une ou plusieurs insaturations.

De préférence, l'acide gras mis en œuvre est un acide palmitique et/ou d'un acide stéarique.

Selon un mode de réalisation avantageux de la présente invention, le procédé met en œ uvre :
- une cire naturelle, avantageusement la cire d'abeille ;
- le polyglycérol ; et
- l'enzyme *candida antarctica lipase B.*

D'une manière générale, la réaction selon le procédé de l'invention peut se faire en masse (c'est-à-dire en absence de solvant) ou en présence de solvant.

Comme exemple de solvant on peut notamment citer : les alcools tels que le tert-butanol ou le DMSO (diméthylsulfoxyde).

Dans tous les cas, que la réaction se fasse en masse ou bien en présence de solvant, elle est de préférence conduite à une température, égale ou supérieure à la température de fusion de la cire. La température de réaction peut notamment être comprise entre 20 et 100°C, plus avantageusement entre 70 et 85°C.

La réaction entre la cire et le polyol est avantageusement réalisée à une température supérieure à la température de fusion de la cire, notamment en l'absence de solvant.

Ces gammes de températures permettent de conserver les qualités organoleptiques, notamment la couleur et l'odeur, des matières premières de départ qui ne sont pas dégradées. Ainsi les cires transformées selon le procédé de l'invention sont des produits de qualité améliorée, ne nécessitant pas d'étapes supplémentaires de purification, par rapport aux excipients obtenus par voie chimique conventionnelle.

De manière générale, la réaction selon le procédé est réalisée pendant une durée avantageusement comprise entre 2 et 24 heures, plus avantageusement entre 6 et 15 heures.

De manière avantageuse, le rapport en masse entre la cire et le polyol est compris entre 99/1 et 80/20, plus avantageusement entre 96/4 et 88/12.

Selon un autre mode de réalisation avantageux, l'enzyme représente 1 à 10% en masse par rapport à la masse de la cire, plus avantageusement 2 à 6% en masse.

De manière générale, le procédé peut être un procédé continu, c'est-à-dire un procédé mettant en œuvre un lit fixe ou fluidisé de catalyseur à travers lequel les produits à transformer circulent.

Il peut également s'agir d'un procédé en discontinu c'est-à-dire un procédé mettant en œuvre un réacteur dans lequel les produits, soumis à une agitation mécanique, restent en contact avec l'enzyme jusqu'à la fin de la réaction.

De manière avantageuse, il s'agit d'un procédé en continu dans lequel l'enzyme est immobilisée et constamment alimentée avec au moins une cire, avantageusement naturelle, et au moins un polyol.

De manière surprenante le Demandeur a constaté que la réaction peut être réalisée, même en l'absence de solvant, alors que la cire hydrophobe et le polyol hydrophile forment un système biphasique. En l'absence de solvant, le procédé est avantageusement réalisé à une température supérieure à la température de fusion de la cire.

L'excipient susceptible d'être obtenu par le procédé décrit ci-avant et qui consiste à faire réagir au moins une cire avec au moins un polyol en présence d'au moins une est utilisé dans une formulation cosmétique.

L'excipient peut notamment représenter entre 0,01 et 99% en masse, de préférence entre 0,1 et 10% en masse de la composition cosmétique.

L'excipient est en général appliqué par voie topique.

L'excipient en général, et les dérivés de cire en particulier, peuvent être formulés sous toutes les formes galéniques normalement utilisées pour une application topique sur la peau ou les cheveux, notamment au sein d'une composition cosmétique sous forme d'un gel huileux, d'une émulsion huile-dans-eau ou eau-dans-huile ou multiple, d'une émulsion siliconée, d'une microémulsion ou nanoémulsion.

La composition peut être plus ou moins fluide et avoir l'aspect entre autre d'une crème blanche ou colorée, d'une pommade, d'un lait, d'une lotion, d'un sérum.

La composition peut contenir les adjuvants habituels dans les domaines cosmétique et dermatologique, tels que les matières grasses, les émulsionnants et co-émulsionnants, les gélifiants hydrophiles ou lipophiles, les actifs hydrophiles ou lipophiles, les conservateurs, les antioxydants, les solvants, les parfums, les charges, les filtres hydrophiles et lipophiles, les matières colorantes, les neutralisants, les agents propénétrants, et les polymères.

Les quantités de ces différents adjuvants sont celles classiquement utilisées dans les domaines considérés, et par exemple de 0,01 à 30% de la masse totale de la composition. Ces adjuvants, selon leur nature, peuvent être introduits dans la phase grasse ou dans la phase aqueuse.

Comme matières grasses utilisables dans la composition cosmétique on peut utiliser les huiles minérales, les huiles d'origine animale (lanoline), les huiles végétales, les huiles de synthèse (isopropyl myristate, octyldodécyl, isostéaryl isostéarate, décyl oléate, isopropyl palmitate), les huiles siliconées (cyclométhicone, diméthicone) et les huiles fluorées. On peut utiliser comme matières grasses des alcools gras, des acides gras, des cires et des gommes et des élastomères de silicone.

Comme émulsionnants et co-émulsionnants utilisables dans la composition cosmétique on peut citer par exemple les esters de polyglycérols et d'acides gras, les esters de saccharose et d'acides gras, les esters de sorbitane et d'acides gras, les esters d'acides gras et de sorbitan polyoxyéthylénés, les éthers d'alcools gras et de PEG, les esters de glycérol et d'acides gras, les alkyl sulfates, les alkyl éther sulfates, les alkyl phosphates, les alkyl polyglucosides, les diméthicones copolyols, et les dérivés de l'acide citrique et de l'acide lactique.

Comme gélifiants hydrophiles, on peut citer en particulier les polymères carboxyvinyliques (carbomer), les copolymères acryliques tels que les copolymères d'acrylates/alkylacrylates, les polyacrylamides, les copolymères d'acide 2-acrylamido-2-méthylpropane, les polysaccharides tels que la gomme xanthane, la gomme guar, la cellulose ses dérivés, les amidons et leurs dérivés, et les argiles.

Comme gélifiants lipophiles, on peut citer les argiles modifiées comme les bentones, les sels métalliques d'acides gras, la silice et ses dérivés et l'éthylcellulose.

La composition cosmétique peut également contenir des actifs. Comme actifs, on peut utiliser notamment les dépigmentants, les émollients, les hydratants, les anti-séborrhéiques, les anti-inflammatoires, les anti-acnéiques, les agents kératolytiques et/ou desquamants, les agents anti-rides et tenseurs, les agents drainants, les agents anti-irritants, les agents apaisants, les amincissants tels que les bases xanthiques (caféine), les vitamines et leurs mélanges, les agents matifiants, les actifs anti-âge tel que le retinol, les agents anti-rides, et les huiles essentielles.

Comme conservateurs utilisables selon l'invention, on peut citer l'acide benzoïque, ses sels et ses esters ; l'acide sorbique et ses sels ; les parabens, leurs sels et esters ; le triclosan ; l'imidazolidinyl urée ; le phenoxyethanol ; la DMDM hydantoïne ; le diazolidinyl urée ; la chlorphenesin.

Comme antioxydants utilisables selon l'invention, on peut citer les agents chélatants tels que l'EDTA et ses sels.

Comme solvants utilisables selon l'invention, on peut citer l'eau, l'éthanol, la glycérine, le propylène glycol, le butylène glycol, le sorbitol, le 1,3-propanediol.

Comme charges utilisables selon l'invention, on peut citer le talc, le kaolin, le mica, la serecite, le magnésium carbonate, l'aluminium silicate, le magnésium silicate, les poudres organiques telles que le nylon.

Comme filtres utilisables selon l'invention, on peut citer les filtres UVA et UVB classiquement utilisés tels que la benzophenone-3, le butyl méthoxydibenzoyl méthane, l'octocrylène, l'octyl méthoxycinnamate, le 4-methylbenzylidene camphor, l'octyl salycylate, le tacephthalydene dicamphor sulfanic acid, et le drométrizole trisiloxane. On citera également les filtres physiques TiO₂ et ZnO sous leurs formes micrométriques et nanométriques, enrobés ou non enrobés.

Comme matières colorantes utilisables selon l'invention, on peut citer les colorants lipophiles, les colorants hydrophiles, les pigments et les nacres habituellement utilisés dans les compositions cosmétiques ou dermatologiques, et leurs mélanges.

Comme neutralisants utilisables selon l'invention, on peut citer la soude, la triéthanolamine, l'aminométhyl propanol, l'hydroxyde de potassium.

Comme agents propénétrants utilisables selon l'invention, on peut citer les alcools et glycols (éthanol, propylène glycol), l'éthoxydiglycol, les alcools et acides gras (acide oléique), les esters d'acides gras, le diméthyl isosorbide.

La composition peut être utilisée comme produit de soin, comme produit de nettoyage, et/ou comme produit de maquillage de la peau, comme produit de protection solaire, ou comme produit capillaire.

A titre d'exemple il peut s'agir d'un rouge à lèvres.

L'invention et les avantages qui en découlent ressortiront mieux des exemples suivants, donnés afin d'illustrer l'invention.

### EXEMPLES DE REALISATION DE L'INVENTION

Les exemples suivants ont pour but de comparer des excipients obtenus par catalyse enzymatique selon l'invention (exemples 1-5), et des excipients obtenus par voie chimique (exemples comparatifs 1-4). L'utilisation des excipients selon l'invention dans différentes formulations cosmétiques est également exemplifiée (exemples 6-12).

On utilise comme cire de départ, la cire d'abeille qui est caractérisée par un indice d'acide = 15,1 mg KOH/g et une couleur Gardner = 1,6.

La quantité de chacun des réactifs est exprimée en parts en masse, pour 100 parts en masse de réactifs.

### Exemple 1 : synthèse d'un excipient par réaction de cire d'abeille et de polyéthylène glycol 400 par catalyse enzymatique

La cire d'abeille (76,19 parts en masse), le PEG400 (19,05 parts en masse) et le catalyseur qui est la lipase immobilisée (*candida antarctica lipase B,* 4,76 parts en masse) sont introduits dans un réacteur. Ce mélange est agité mécaniquement et maintenu à pression atmosphérique. Le réacteur est chauffé à 80°C pendant 24 heures. Le milieu réactionnel est ensuite filtré pour retirer le catalyseur. Le filtrat est décanté à chaud pour éliminer le PEG en excès. La cire transformée est ensuite filtrée afin d'éliminer toute impureté solide.

Le rendement de la réaction est 65%, par rapport à la quantité totale initiale de réactifs. Le rendement correspond au rapport entre la masse de produit récupéré et la masse des produits engagés au départ
Mesure de l'indice d'acide en fin de réaction = 1,1mg KOH/g

### Exemple 2 : recyclage du catalyseur enzymatique

La lipase immobilisée de l'exemple 1 est récupérée après filtration et est réutilisée. Dans cet exemple, il est montré que la lipase supportée peut être réutilisée ou recyclée.

Le protocole expérimental est le même que celui de l'exemple 1.

Mesure de l'indice d'acide en fin de réaction = 3,1mg KOH/g.

### Exemple comparatif 1 : Synthèse d'un excipient par réaction de cire d'abeille et de polyéthylène glycol 400 par catalyse chimique

Les réactifs, PEG 400 (19,92 parts en masse), la cire d'abeille (79,66 parts en masse) et la soude (0,24 parts en masse) sont introduits dans un réacteur agité équipé d'une régulation thermique et d'un système d'introduction d'azote pour protéger de l'oxydation.

L'ensemble est chauffé à 215°C pendant 7 heures. Le milieu réactionnel est neutralisé à l'acide (0,18 parts en masse de H₃PO₄). Le contenu du réacteur est ensuite décanté pour éliminer le PEG en excès. La phase supérieure contenant le dérivé de cire (excipient) est filtrée à chaud puis conditionnée.

Le rendement de la réaction est 90%, par rapport à la quantité totale initiale de réactifs. Le rendement correspond au rapport entre la masse de produit récupéré et la masse des produits engagés au départ.

La valeur de l'indice d'acide en fin de réaction est 1,9 mg KOH/g.
Couleur Gardner = 4,4

### Exemple comparatif 2 : réalisation d'un blanc, sans catalyse

L'exemple 1 est reproduit, sans catalyseur (20 parts en masse de cire d'abeille et 80 parts en masse de PEG 400), afin de réaliser un blanc.

Le rendement de la réaction est 75%, par rapport à la quantité totale initiale de réactifs. Le rendement correspond au rapport entre la masse de produit récupéré et la masse des produits engagés au départ.

Mesure de l'indice d'acide en fin de réaction = 13,1 mg KOH/g.

En comparant les réactions des exemples 1 et 2, on constate que la cire a bien été transformée. A la différence des exemples 1 et 2, la filtration n'est pas nécessaire puisque le catalyseur à éliminer (e.g. lipase immobilisée) est absent.

La composition des produits de synthèse des exemples 1 et 2 et des exemples comparatifs 1 et 2 est déterminée et détaillée dans le tableau 1 ci-dessous. Les analyses consistent à doser les esters de PEG et les alcools gras libérés lors de la synthèse.

La composition en esters de PEG et en PEG libre est déterminée par chromatographie liquide HPLC-ELSD (High Pressure Liquid Chromatography - Evaporative Light Scattering Detector) après une préparation d'échantillon par extraction en phase solide.

La composition en alcools gras libres est déterminée par chromatographie en phase gazeuse couplée à un détecteur à ionisation de flamme GC-FID après silylation avec du héxaméthyldisilazane combiné avec du triméthylchlorosilane (HMDS-TMCS). De manière générale, les alcools gras ont des chaînes grasses allant de 16 à 34 carbones.

**Tableau 1 : analyse des produits de synthèse des exemples 1 et 2 et des exemples comparatifs 1 et 2.**

| | exemple 1 | exemple 2 | exemple comparatif 1 | exemple comparatif 2 | cire d'abeille |
|---|---|---|---|---|---|
| catalyseur | enzyme | enzyme recyclée | NaOH | - | - |
| alcools gras libres | 4,1% | 3,4% | 4,5% | 0,7% | 0,8% |
| acides gras libres | 0,5% | 1,3% | 0,8% | 5,6% | 6,5 |
| monoesters de PEG | 4,5% | 6,6% | 12,5% | 1,8% | - |
| diesters de PEG | 6,7% | 5,5% | 5,2% | 0,3% | - |
| PEG libre | 0,5% | 0,9% | 3,7% | 0,4% | - |

Le reste de la composition est constituée d'esters aliphatiques.

On voit dans le tableau que la lipase catalyse la réaction de formation des monoesters et diesters de PEG par une réaction d'estérification (diminution de l'acidité libre), mais catalyse aussi la réaction de transestérification (libération d'alcools gras libres). Cependant la teneur en diesters est différente de celle obtenue par voie chimique.

La composition des produits obtenus par les deux types de procédés n'est pas la même.

### Exemple 3: Synthèse d'un dérivé de cire d'abeille en présence de polyglycérol-3 par catalyse enzymatique

La cire d'abeille (85,7 parts en masse), le polyglycérol-3 (9,5 parts en masse) et le catalyseur (*candida antarctica lipase B,* immobilisée ; 4,8 parts en masse) sont introduits dans un réacteur agité mécaniquement et maintenu à pression atmosphérique sous bullage d'azote. Le réacteur est chauffé à 80°C pendant 24 heures. Le milieu réactionnel est ensuite filtré grossièrement pour retirer le biocatalyseur. Le filtrat est décanté à chaud pour éliminer le polyglycérol-3 en excès. La cire transformée est ensuite filtrée finement afin d'éliminer toute impureté solide.

Le rendement de la réaction est 68,6%, par rapport à la quantité totale initiale de réactifs. Mesure de l'indice d'acide en fin de réaction = 0,51 mg KOH/g.
Couleur Gardner = 1,9

### Exemple 4 : Synthèse d'un dérivé de cire d'abeille en présence d'acides gras par catalyse enzymatique

On peut aussi associer des acides gras avec la cire, en présence de polyglycérol-3, pour obtenir plus d'esters de polyglycérol, ces derniers sont utilisés comme émulsifiants dans les applications cosmétiques.

La cire d'abeille (83,4 parts en masse), les acides gras (acides stéarique et palmitique ; 2,8 parts en masse), le polyglycérol-3 (9,3 parts en masse) et le catalyseur (*candida antarctica lipase B,* immobilisée ; 4,6 parts en masse) sont introduits dans un réacteur agité mécaniquement et maintenu à pression atmosphérique sous bullage d'azote. Le réacteur est chauffé à 80°C pendant 24 heures. Le milieu réactionnel est ensuite filtré pour retirer le catalyseur. Le filtrat est décanté à chaud pour éliminer le polyglycérol-3 en excès. Le milieu réactionnel est ensuite filtré afin d'éliminer toute impureté solide.

Le rendement de la réaction est 64,5%, par rapport à la quantité totale initiale de réactifs.

Mesure de l'indice d'acide final = 0,31 mg KOH/g.
Couleur Gardner = 1,9

### Exemple comparatif 3 : synthèse d'un dérivé de cire d'abeille en présence d'acides gras en utilisant un catalyseur chimique

La cire d'abeille (83,4 parts en masse), les acides gras (acides stéarique et palmitique ; 2,8 parts en masse) et le polyglycérol-3 (9,3 parts en masse) sont introduits dans un réacteur agité. De l'azote est introduit pour éviter l'oxydation. La réaction est catalysée à la soude (0,24 parts en masse). La température de la réaction est fixée à 215°C pendant 7 heures. En fin de réaction, le mélange réactionnel est neutralisé avec de l'acide phosphorique (0,18 parts en masse). Le contenu du réacteur est ensuite décanté pour éliminer le polyglycérol-3 insoluble en excès. Le produit est filtré à chaud puis conditionné.

Le rendement de la réaction est 84,3%, par rapport à la quantité totale initiale de réactifs.

Mesure de l'indice d'acide final = 1,13 mg KOH/g.
Couleur Gardner = 6,9

Les produits sont analysés comme suit :
Injection GC-FiD après dérivation au BSTFA pour quantifier les alcools gras libres (standard interne alcool en C19), les alcanes et les alcènes (standard interne : squalane).
Injection GC-FiD du produit brut pour déterminer la quantité d'esters aliphatiques (standard interne : ester aliphatique en C41).

Le reste de la composition est constitué des esters de polyglycérol.

**Tableau 2 : comparatif de la synthèse par voie chimique et enzymatique.**

| | exemple 3 | exemple 4 | exemple comparatif 3 |
|---|---|---|---|
| Catalyseur | Enzyme | Enzyme | NaOH |
| couleur | 1,9 | 1,9 | 6,9 |
| alcools gras libres | 0,9% | 0,7% | 5,7% |
| acides gras libres | 0,5% | 0,13 | 1,13% |
| alcanes + alcènes | 13,3% | 13,0% | 12,6% |
| esters aliphatiques | 61,1% | 57,8% | 41,9% |
| esters de polyglycérol | 24,2% | 28,4% | 38,7% |

On constate que la lipase a bien catalysé la formation des esters de polyglycérol de la cire en présence ou non d'acides gras. La teneur en alcools gras est plus importante dans la réaction catalysée par la soude. La composition des produits est donc différente en fonction du procédé employé.

### Exemple 5 : synthèse d'un dérivé de cires naturelles (liquide et solide) par catalyse enzymatique

La cire d'abeille (34,52 parts en masse), l'huile de jojoba (58,75 parts en masse), le polyglycérol-3 (1,95 parts en masse) et la lipase immobilisée (*candida antarctica lipase B*, immobilisée ; 4,78 parts en masse) sont introduits dans un réacteur agité mécaniquement et maintenu à pression atmosphérique sous un bullage d'azote. Le réacteur est chauffé à 80°C pendant 24 heures. Le milieu réactionnel est ensuite filtré grossièrement pour retirer la lipase. Le filtrat est décanté à chaud pour éliminer le polyglycérol-3 en excès. La phase supérieure, contenant le dérivé de cires est ensuite filtrée finement afin d'éliminer toute impureté solide.

Le rendement de la réaction est 78,3%, par rapport à la quantité totale initiale de réactifs.
Mesure de l'indice d'acide final = 1,82 mg KOH/g
Couleur Gardner = 2,1

### Exemple comparatif 4 : synthèse d'un dérivé de cires naturelles (liquide et solide) par catalyse par voie chimique

Synthèse d'un dérivé de cires naturelles selon le procédé décrit dans le document FR 2 891 736 B1.

| | parts en masse |
|---|---|
| polyglycérol-3 | 2 |
| huile de jojoba | 61,8 |
| cire d'abeille | 36,1 |
| NaOH | 0,0745 |
| H₃PO₄ 85% | 0,1863 |
| terre filtrante | 0,25 |
| Rendement | 80,0% |

Indice d'acide en fin de réaction = 1,08 mg KOH/g
Mesure de la couleur Gardner = 3,7

**Tableau 3 : détermination de la composition des produits issus de l'exemple 5 et de l'exemple comparatif 4.**

| | exemple 5 | exemple comparatif 4 |
|---|---|---|
| Catalyseur | Enzyme | NaOH |
| couleur | 2,1 | 3,7 |
| alcools gras libres | 1,2% | 4,3% |
| acides gras libres | 1,8% | 1,1 |
| alcanes + alcènes | 4,7% | 5,5% |
| esters aliphatiques | 80,3% | 72,7% |
| esters de polyglycérol | 12,0% | 16,4% |

Ce tableau montre l'effet avantageux de la catalyse enzymatique selon l'invention. Le dérivé de cire obtenu selon l'invention (exemple 5) présente une couleur plus proche de celle de la cire d'abeille que le dérivé préparé selon l'art antérieur décrit dans le document FR 2 891 736 B1 (exemple comparatif 4).

Les exemples 6-12 suivants montrent des utilisations potentielles des produits des exemples précédents dans les applications cosmétiques. Les quantités respectives des ingrédients sont exprimées en pourcentage en masse.

### Exemple 6 : émulsion huile dans eau, comprenant l'excipient selon l'exemple 1

| **Ingrédients** | % |
|---|---|
| eau déminéralisée | 62,85 |
| carbopol Ultrez 21 (acrylates/C10-30 alkyl acrylate crosspolymer) | 0,2 |
| Glycerine | 5 |
| gomme Xanthane | 0,2 |
| exemple 1 | 3 |
| emulcire 61 (cetyl alcool, ceteth-20, steareth-20) | 2 |
| lipocire A (C10-18 triglycerides) | 3 |
| geleol (glyceryl stearate) | 0,5 |
| MOD (octyldodecyl Myristate) | 6 |
| labrafac CC (caprylic/capric triglycerides) | 10 |
| DPPG (propylene glycol dipelargonate) | 6 |
| phenonip (phenoxyethanol, methylparaben, ethylparaben, butylaraben, propylparaben, | 0,8 |
| solution aqueuse à 10% NaOH | 0,45 |

### Exemple 7: Formulation pour rouge à lèvres, comprenant l'excipient selon l'exemple 3

| **Phase** | **Ingrédients** | % |
|---|---|---|
| A | cire d'abeille | 8,0 |
| | cire de carnauba | 6,5 |
| | cire de candelilla | 5,5 |
| | lipocire A (C10-18 triglycérides) | 2,0 |
| | huile de jojoba | 10,0 |
| | MOD (myristate d'octyldodécyle) | 32,5 |
| | isostéarate isostéaryle | 20,0 |
| | unipure red LC 388 (oxydes de fer) | 6,0 |
| | unipure pink LC583 (violet de manganèse) | 4,0 |
| B | exemple 3 | 3,0 |
| | extrait originel cerise bio | 2,0 |
| C | tocophérol | 0,1 |
| | parfum | 0,4 |

### Exemple 8: Formulation gel huileux (baume anhydre), comprenant l'excipient selon l'exemple 3

| **Phase** | **Ingrédients** | % |
|---|---|---|
| A | COMPRITOL® 888 (glyceryl behenate) | 7,5 |
| | exemple 3 | 7,5 |
| | LABRAFAC CC (caprylic/capric triglycerides) | 45,0 |
| | huile de jojoba | 15,0 |
| | octyldodecyl myristate | 10,0 |
| | cetiol®C5 (coco-caprylate-) | 15,0 |

### Exemple 9: Emulsion huile dans eau (crème de jour), comprenant l'excipient selon l'exemple 3

| **Phase** | **Ingrédients** | % |
|---|---|---|
| A | eau déminéralisé | 67,95 |
| | E.D.T.A. sel disodique | 0,10 |
| | Carbopol® Ultrez 10 (carbomer) | 0,15 |
| B | glycérine | 3,00 |
| | Satiaxane® CX91 (gomme xanthane) | 0,20 |
| C | Emulium® Delta Hexadécanol, glycéryl stéarate, PEG-75 stéarate, ceteth-20, steareth-20 | 4,00 |
| | exemple 3 | 2,00 |
| | Parsol® MCX, ethylhexylméthoxycinnamate | 6,00 |
| | Parsol®340 (octocrylene ; CAS : 6197-30-4) | 2,00 |
| | Parsol® 1789 (butylméthoxydibenzoylméthane). | 3,00 |
| | Cetiol® C5 (coco-caprylate) | 2,00 |
| | Cocoate BG (butylène glycol cocoate) | 2,00 |
| | Cetiol® Sensoft (propylheptyl caprylate) | 4,00 |
| | conservateurs | 1,00 |
| D | solution aqueuse de soude à 10% | 0,30 |
| E | Dry-Flo® PLUS (CAS : 9087-61-0) | 2,00 |
| | parfum | 0,30 |

### Exemple 10: Emulsion huile dans eau (crème E/H), comprenant l'excipient selon l'exemple 3

| **Phase** | **Ingrédients** | % |
|---|---|---|
| A | Plurol® Diisostéarique CG (polyglycéryl-3 diisostéarate) | 3,00 |
| | exemple 3 | 2,00 |
| | Compritol® 888 CG pastilles (glycéryl béhénate). | 1,00 |
| | huile de ricin hydrogénée (CAS : 8001-78-3) | 2,25 |
| | Cetiol® CC (dicaprylyl carbonate). | 22,00 |
| | Squalane végétal | 3,00 |
| | Talc LCW (Talc) | 5,00 |
| B | eau déminéralisée | 57,45 |
| | sulfate de magnésium, 7 H₂O, Codex | 1,50 |
| | NaCl, CODEX | 1,50 |
| C | conservateur | 1,00 |
| D | Perfume Supreme TeintARX/31619 (parfum) | 0,30 |

### Exemple 11: Formulation pour maquillage, mascara, comprenant l'excipient selon l'exemple 3

| **Phase** | **Ingrédients** | % |
|---|---|---|
| A | exemple 3 | 4,0 |
| | cire de carnauba | 3,0 |
| | cire de paraffine | 12,0 |
| | Geleol® (stéarate de glycérol CAS : 31566-31-1) | 3,0 |
| | acide stéarique | 6,0 |
| B | AMP Ultra PC 2000 (aminométhyl propanol) | 0,5 |
| C | UNIPURE TRIPLE BLACK (oxyde de fer noir) | 6,0 |
| D | eau déminéralisée | 64,0 |
| | Natrosol® 250 HHX Pharma (Hydroxyéthylcellulose) | 0,5 |
| E | SATIAXANE CX91 (gomme xanthane) | 0,2 |
| | Phenonip (phénoxyéthanol, méthylparaben, butylparaben, propylparaben) | 0,8 |

### Exemple 12: Formulation pour gel crème (Bi-gel), comprenant l'excipient selon l'exemple 3

| **Phase** | **Ingrédients** | % |
|---|---|---|
| A | eau déminéralisée | 74,05 |
| | Carbopol® Ultrez 10 (carbomer) | 0,25 |
| | Glycérine | 2,00 |
| | Satiaxane CX91 (gomme xanthane) | 0,20 |
| B | Emulfree® CBG (alcool isostéarylique, cocoate de butylène glycol, éthylcellulose) | 2,00 |
| | exemple 3 | 3,00 |
| | Lipocire® A (triglycérides C10-C18) | 2,00 |
| | Hexadécanol | 2,00 |
| | Cetiol® CC (dicaprylyl carbonate) | 13,00 |
| | phénoxyéthanol | 0,63 |
| | ethylhexylglycérine | 0,07 |
| C | solution aqueuse de soude à 10% | 0,50 |
| D | Perfume FlavoratonicCRE/1193 (Parfum) | 0,30 |

## Revendications

1. Utilisation comme excipient dans une formulation cosmétique, du produit obtenu par réaction d'au moins une cire avec au moins un polyol en présence d'au moins une lipase choisie dans le groupe constitué par : *Candida antarctica lipase A ; Candida antarctica lipase B ; Candida rugosa ; Thermomyces lanuginosus ; Rhizomucor miehei ; Pseudomonas sp.* ; et *Carica papaya,*
la cire étant une cire naturelle ou une cire synthétique,
la cire naturelle étant d'origine végétale, animale ou minérale,
la cire synthétique étant un mélange d'esters d'acides gras et d'alcools gras obtenus par estérification.

2. Utilisation selon la revendication 1, ***caractérisée* en ce que** la lipase est supportée sur un substrat solide.

3. Utilisation selon l'une des revendications 1 à 2, ***caractérisée* en ce que** la cire est une cire naturelle choisie dans le groupe constitué par : cire d'abeille ; cire de candelilla ; cire de carnauba ; cire de tournesol ; cire de son de riz ; cire de canne à sucre ; cire de jojoba ; cire de mimosa ; cire d'olive ; et cire de shellac.

4. Utilisation selon l'une des revendications 1 à 3, ***caractérisée* en ce que** le polyol est choisi dans le groupe constitué par l'éthylène glycol ; diéthylène glycol ; triéthylène glycol ; polyéthylène glycol ; 2-méthyl propanediol ; propylène glycol ; polypropylène glycol ; butylène glycol ; néopentyl glycol ; hexylène glycol ; octylène glycol ; triméthylol propane ; sorbitol ; érythritol ; pentaerythritol ; dipentaerythritol ; glycérol, diglycérol ; polyglycérol ; isosorbide ; sorbitan ; polytriméthylène éther glycol ; et 1,3-propanediol.

5. Utilisation selon l'une des revendications 1 à 4, ***caractérisée* en ce que** la réaction est réalisée également en présence d'au moins un acide gras.

6. Utilisation selon l'une des revendications 1 à 5, ***caractérisée* en ce que** :
- la cire est la cire d'abeille,
- le polyol est le polyglycérol ;
- la lipase est *candida antarctica lipase B.*

7. Utilisation selon l'une des revendications 1 à 6, ***caractérisée* en ce que** la réaction est réalisée à une température comprise entre 20 et 100°C, avantageusement entre 70°C et 85°C.

8. Utilisation selon l'une des revendications 1 à 7, ***caractérisée* en ce que** le rapport en masse cire/polyol est compris entre 99/1 et 80/20, avantageusement entre 96/4 et 88/12.

9. Utilisation selon l'une des revendications 1 à 8, ***caractérisée* en ce que** la lipase représente 1 à 10% en masse par rapport à la masse de la cire.

10. Utilisation selon l'une des revendications 1 à 9, ***caractérisée* en ce que** l'excipient est obtenu en l'absence de solvant, et **en ce que** la réaction est réalisée à une température supérieure à la température de fusion de la cire.

## Patentansprüche

1. Einsatz als Trägerstoff in einer kosmetischen Rezeptur eines Produktes, das Ergebnis ist der Reaktion mindestens eines Wachses mit mindestens einem Polyol, in Anwesenheit mindestens einer Lipase, ausgewählt aus der Gruppe bestehend aus:
*Candida antarctica lipase A; Candida antarctica lipase B; Candida rugosa; Termomyces lanuginosus; Rhizomucor miehei; Pseudomonas sp. und Caricapapaya,*
bei dem Wachs handelt es sich dabei um ein natürliches Wachs oder ein synthetisches Wachs,
das natürliche Wachs ist dabei pflanzlicher, tierischer oder mineralischer Herkunft, bei dem synthetischen Wachs handelt es sich dabei um eine Mischung aus Fettsäuren und Fettalkoholen, erhalten durch Veresterung.

2. Einsatz nach Anspruch 1, ***dadurch gekennzeichnet, dass*** die Lipase von einem festen Substrat getragen wird.

3. Einsatz nach einem der Ansprüche 1 bis 2, ***dadurch gekennzeichnet, dass*** es sich bei dem Wachs um ein natürliches Wachs handelt, ausgewählt aus der Gruppe bestehend aus: Bienenwachs, Candelillawachs, Carnaubawachs, Sonnenblumenwachs, Reiskleiewachs, Zuckerrohrwachs, Jojoba- Wachs, Mimosenwachs, Olivenwachs und Schellackwachs.

4. Einsatz nach einem der Ansprüche 1 bis 3, ***dadurch gekennzeichnet, dass*** das Polyol ausgewählt wird aus der Gruppe bestehend aus: Ethylenglycol; Diethylenglycol; Triethylenglycol; Polyethylenglycol; 2-methyl-propandiol; Propylenglycol; Polypropylenglycol; Butylenglycol; Neopentylglycol; Hexylenglycol; Oktylenglycol; Trimethylol - Propan; Sorbitol; Erythritol; Pentaerythritol; Dipentaerythritol, Glyzerin, Diglyzerin, Polyglyzerin; Isosorbid; Sorbitan; Polytrimethylenetherglycol; und 1,3 - Propandiol.

5. Einsatz nach einem der Ansprüche 1 bis 4, ***dadurch gekennzeichnet, dass*** die Reaktion in Anwesenheit auch mindestens einer Fettsäure durchgeführt wird.

6. Einsatz nach einem der Ansprüche 1 bis 5, ***dadurch gekennzeichnet, dass***
- es sich bei dem Wachs um Bienenwachs handelt,
- das Polyol Polyglyzerin ist;
- es sich bei der Lipase um *candida antarctica lipase B* handelt.

7. Einsatz nach einem der Ansprüche 1 bis 6, ***dadurch gekennzeichnet, dass*** die Reaktion bei einer Temperatur zwischen 20 und 100° C, durchgeführt wird, vorteilhafterweise zwischen 70° C und 85° C.

8. Einsatz nach einem der Ansprüche 1 bis 7, ***dadurch gekennzeichnet, dass*** das Gewichtsverhältnis Wachs/Polyol zwischen 99/1 und 80/20 liegt, vorteilhafterweise zwischen 96/4 und 88/12.

9. Einsatz nach einem der Ansprüche 1 bis 8, ***dadurch gekennzeichnet, dass*** die Lipase 1 bis 10 Gewichts-% bezogen auf das Gewicht des Wachses darstellen.

10. Einsatz nach einem der Ansprüche 1 bis 9, ***dadurch gekennzeichnet, dass*** der Trägerstoff ohne Lösungsmittel erhalten wird und dass die Reaktion bei einer Temperatur über der Schmelztemperatur des Wachses stattfinde.

## Claims

1. Use, as an excipient in a cosmetic formulation, of the product obtained by reaction of at least one wax with at least one polyol in the presence of at least one lipase selected from the group constituted by: *Candida antarctica lipase A; Candida antarctica lipase B; Candida rugosa; Thermomyces lanuginosus; Rhizomucor miehei; Pseudomonas sp.;* and *Carica papaya,*
the wax being a natural wax or a synthetic wax,
the natural wax being of animal, vegetable or mineral origin,
the synthetic wax being a mixture of esters of fatty acids and of fatty alcohols obtained by esterification.

2. Use as claimed in claim 1, ***characterized in that*** the lipase is supported on a solid substrate.

3. Use as claimed in claim 1 or claim 2, ***characterized in that*** the wax is a natural wax selected from the group constituted by: beeswax; candelilla wax; carnauba wax; sunflower wax; rice bran wax; sugar cane wax; jojoba wax; mimosa wax; olive wax; and shellac wax.

4. Use as claimed in one of claims 1 to 3, ***characterized in that*** the polyol is selected from the group constituted by: ethylene glycol; diethylene glycol; triethylene glycol; polyethylene glycol; 2-methyl propanediol; propylene glycol; polypropylene glycol; butylene glycol; neopentyl glycol; hexylene glycol; octylene glycol; trimethylol propane; sorbitol; erythritol; pentaerythritol; dipentaerythritol; glycerol, diglycerol; polyglycerol; isosorbide; sorbitan; polytrimethylene ether glycol; and 1,3-propanediol.

5. Use as claimed in one of claims 1 to 4, ***characterized in that*** the reaction is also carried out in the presence of at least one fatty acid.

6. Use as claimed in one of claims 1 to 5, ***characterized in that:***
- the wax is beeswax,
- the polyol is polyglycerol;
- the lipase is candida antarctica lipase B.

7. Use as claimed in one of claims 1 to 6, ***characterized in that*** the reaction is carried out at a temperature in the range 20°C to 100°C, advantageously in the range 70°C to 85°C.

8. Use as claimed in one of claims 1 to 7, ***characterized in that*** the ratio by weight of wax/polyol is in the range 99/1 to 80/20, advantageously in the range 96/4 to 88/12.

9. Use as claimed in one of claims 1 to 8, ***characterized in that*** the lipase represents 1% to 10% by weight with respect to the mass of the wax.

10. Use as claimed in one of claims 1 to 9, ***characterized in that*** the excipient is obtained in the absence of solvent, and **in that** the reaction is carried out at a temperature which is above the melting temperature of the wax.
